# EUROPEAN PATENT APPLICATION

(11) **EP 0 766 973 A1**
(43) Date of publication of application: **09.04.1997**
(21) Application number: 96306554.5
(22) Date of filing: 10.09.1996
(51) Int. Cl.: A61L 33/00, A61B 5/14

(54) **Blood collection device for plasma separation and method therefor**

(30) Priority: 29.09.1995 US 535424
(71) Applicant: Becton Dickinson and Company, Franklin Lakes, New Jersey 07417-1880 (US)
(72) Inventor: Sydney, Gregory T., Sharon, Massachusetts (US); Losada, Robert J., Astoria, New York (US)
(74) Representative: Ruffles, Graham Keith

(57) **Abstract**

A blood collection assembly includes an evacuated plastic tube having an open end with a puncturable stopper therein. A thixotropic gel for separating blood components is in the tube and a layer of heparin particles is spray dried onto the inside tube wall. The invention includes a method to make the tube.

## Description

### BACKGROUND OF THE INVENTION

1. Field of the Invention. This invention relates to blood collection and more particularly relates to a sample collection tube containing a particular anticoagulant and to a method of preparing the tube.

2. Background. Blood samples are routinely taken in evacuated tubes. One end of a double-ended needle is inserted into a patient's vein. The other end of the needle then punctures a septum covering the open end of the tube so that the vacuum in the tube draws the blood sample through the needle into the tube. Using this technique, a plurality of samples can be taken using a single needle puncture of the skin.

Blood drawn into a tube is typically mixed with an additive present in the tube prior to draw. Clot activators such as silica particles promote rapid coagulation so that the liquid serum fraction can be readily separated from the clotted cells. Anticoagulants are used to prevent clotting when the blood sample is to be used directly in hematological tests or for separation of blood cells from the plasma. For separation, the blood sample is centrifuged, and a gel having aspecific gravity between that of the solid and liquid fractions is used as a barrier between the fractions.

Collection tubes are conventionally made of glass or plastic, such as polyethylene terephthalate (PET). Glass tubes have the advantage of water and gas impermeability. Plastic tubes are advantageous over glass in lower breakage, less weight in shipment and easier disposal by incineration, but high permeability to water and gas is a disadvantage.

In glass it has been conventional to provide the anticoagulant in aqueous solution to provide rapid mixing with the blood. However, the action of a liquid additive in the tube may be masked by the gel. If the additive is applied to the wall of the glass tube, it rapidly runs down the tube wall and collects in the bottom of the tube where, again, its action may be masked by the gel. For this reason the tube wall is often coated with polyvinylpyrrolidone (PVP) and the anticoagulant solution applied thereover. The PVP dissolves in the blood and becomes part of the plasma.

Aqueous formulations are unsatisfactory in plastic because permeation through the tube wall changes additive concentration, may reduce additive functionality and may reduce shelf life. A recent approach to providing additives in plastic tubes is disclosed in US Patent No. 5,213,765 to Kasai wherein a glass or plastic blood collection tube contains a plastic film insert having an anticoagulant affixed thereto. A disadvantage of Kasai is that the film becomes part of the packed cells and can interfere with analyses which use the cells, such as blood typing.

There is a need for a plastic blood collection tube which provides heparin anticoagulant in solid form which will readily dissolve when blood enters the tube and yet leave both the plasma and cell fractions free of extraneous materials. The present invention fulfills this need.

### SUMMARY OF THE INVENTION

A blood collection assembly includes a plastic tube having a bottom wall continuous with a side wall. The side wall defines an open end and the bottom wall defines a closed end. Together the bottom and side walls define an inside wall surface. Preferably, the open end is covered by a puncturable stopper and the tube is evacuated. A conventional gel is stored in the bottom of the tube. The inside wall surface is coated with a layer of spray dried anticoagulant particles.

A second aspect of the invention is a method to make the tube.

The spray dried coating of anticoagulant particles is applied from a water solution and eliminates the PVP vehicle used to coat glass tubes with additive. The anticoagulant is dry and particulate thereby preventing any changes in the additive during shelf time and providing a large surface area for rapid dissolution in the blood.

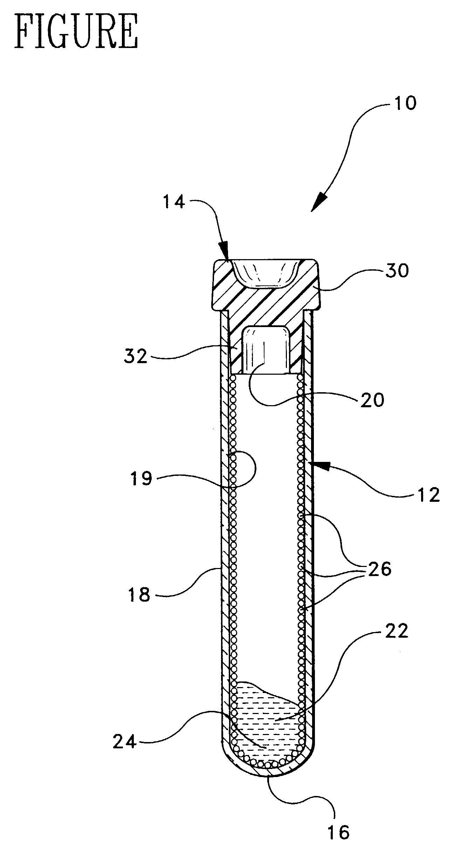

### BRIEF DESCRIPTION OF THE DRAWING

The Figure is a vertical sectional view of the tube of the invention showing the particulate nature of the additive.

### DETAILED DESCRIPTION

While this invention is satisfied by embodiments in many different forms, there will herein be described in detail preferred embodiments of the invention with the understanding that the present disclosure is to be considered as exemplary of the principles of the invention and is not intended to limit the invention to the embodiments illustrated and described. The scope of the invention will be measured by the appended claims and their equivalents.

The blood collection assembly of the invention may include any plastic container having a closed end and an open end. Suitable containers are, for example bottles, vials, flasks and the like, preferably tubes. The invention will henceforth be described in terms of the preferred tube and the preferred anticoagulant, heparin.

Adverting now to the drawing, the Figure illustrates a blood collection assembly 10 which includes a tube 12 and a puncturable stopper 14. Tube 12 has a bottom wall 16 and a side wall 18 having an inside wall surface 19. Side wall 18 defines an open end 20 into which the stopper 14 may be placed. Bottom wall 16, side wall 18 and stopper 14 enclose an interior volume 22 of the tube which contains a conventional gel barrier material 24 and preferably is evacuated. A layer 26 of heparin particles, preferably a salt such as sodium or lithium heparin, is spray dried onto inside wall surface 19. While the drawing shows the particles to cover substantially all of inside wall surface 19, it is only necessary that a fraction of wall surface 19 be covered.

Stopper 14 may include an annular upper portion 30 which extends over the top edge of side wall 18 and a lower annular portion or skirt 32 which extends into and forms an interference fit with inside wall surface 19 for maintaining stopper 14 in place in open end 20. The invention is not limited to the stopper design shown in the drawing.

The tube of the invention is plastic. Suitable plastics are PET, polypropylene and polystyrene. While the tube may be of any size, the invention is particularly well suited to evacuated blood collection tubes. These tubes are generally cylindrical, 50 to 150mm in length and about 10 to 20mm in diameter. The stopper may be of any elastomer, as is well known in the art of evacuated blood collection tubes.

Any conventional gel as known in the art for separating serum or plasma from a cellular fraction may be used. A preferred gel is a thixotropic polyester gel.

While heparin, particularly lithium heparin, is the preferred anticoagulant, other conventional anticoagulants, such as sodium citrate or ethylenediaminetetraacetic acid may be used. Any solvent which dissolves the anticoagulant may be used, preferably water.

The lithium heparin may preferably be applied by spraying a mist of an aqueous solution to the inside wall surface of the plastic tube and drying by a current of warm air to leave a coating or layer of solid particles. Any other method for applying the heparin solution and drying which results in discrete particles may be used.

In accordance with the invention it has been found that the mist "beads up" into discrete droplets which remain in place on the plastic surface and, on drying, leaves a coating of individual particles. In contrast, when an aqueous mist is applied to a hydrophilic glass surface, a fully wetted surface results, the mist runs down the inside wall of the tube and, after drying, only a thin continuous film of low surface area of anticoagulant remains.

Any quantity of heparin which prevents clotting may be used, generally determined by the draw volume. While not wishing to be limited thereby, about 50 to 250 IU units of heparin may be applied. The solution to be misted may be of any concentration. In practice, it has been found that a solution containing about 8000 to 10000 IU units per ml is satisfactory.

### EXAMPLE I

### Application of Additive to PET tube.

The inside wall surface of a 16 X 100mm PET tube was misted with an aqueous solution of lithium heparin so that 192 units of heparin were uniformly dispersed over the tube wall. In addition, 13 X 75mm tubes and 13 X 100mm tubes were misted with 72 units and 108 units per tube respectively. Spraying was performed using an ultrasonic spray nozzle and positive displacement dispenser. The tubes were dried by blowing hot air through a convection nozzle inserted into the tube. A conventional thixotropic polyester gel was then added to the bottom of the tube using a positive displacement dispenser and a nozzle. The tube was held at a 45° angle for three hours to allow the gel to set, sterilized with gamma irradiation at 2.5 mrads, stoppered and evacuated.

### EXAMPLE II

Tubes (16 X 100 mm) of Example I were compared for efficacy with a conventional commercial glass tube (the Control Tube) having a coating of lithium heparin on PVP.

Blood was collected from 20 normal donors into 1 Control Tube and 1 Test Tube from a single venipuncture according to a randomization schedule. Immediately after filling, the tubes were inverted 10 times and centrifuged within 70 minutes of collection at 1000 RCF for 10 minutes at 25(±2)°C. Following centrifugation, all tubes were visually inspected for complete barrier formation, fibrin and hemolysis. A plasma sample from each tube was then placed into a sample cup and analyzed on the Kodak Ektachem^{TM}) 250 Chemistry Analyzer for Chemistry Analytes. A sample size of 20 was used to achieve a power of 0.92 to detect differences of 1.25 S.D. at P=0.01. A total of 18 analytes were studied.

Results were grouped by analyte and the means and standard deviations computed for each tube type. The individual results from the specimens collected into the Test Tubes were differenced with the individual results of specimens collected into the Control Tubes. Paired t values were calculated for each analyte to establish equivalency between tube types.

### Solubility Study:

Deionized water was drawn into the Test Tubes, the Tubes were inverted 5 times, the stoppers were removed and the water poured out. The interior of the tube walls were examined and found to be free of the spray dried lithium heparin.

### Visual Observations

None of the tubes, Test or Control, showed visual hemolysis of the specimens or fibrin and all gel barriers for all tubes were completely formed.

### Statistical Analysis

No statistically significant differences were detected when comparing the Test Tube results with the Control Tube results for equivalency of standard chemistry analytes.

## Claims

1. A blood collection assembly comprising a plastic container having a bottom wall providing a closed end, a side wall defining an open end, and a gel in said closed end, said side wall and bottom wall together defining an inside wall surface, at least a portion of said inside wall surface having a layer of solid anticoagulant particles affixed thereto.

2. The assembly of Claim 1 having a puncturable stopper in said open end.

3. The assembly of Claim 1 in which said container is evacuated.

4. A blood collection assembly comprising:
a) an evacuated plastic tube having a bottom wall providing a closed end and a side wall defining an open end, said side wall and bottom wall together defining an inside wall surface;
b) a puncturable stopper in said open end;
c) a spray-dried coating of discrete particles of a heparin salt on said inside wall surface; and
d) a thixotropic gel in said tube.

5. A method for coating a blood collection tube comprising applying a solution of an anticoagulant to the inside wall surface of a plastic tube and drying said wall surface to leave a coating of anticoagulant particles on said wall surface.

6. The method of Claim 5 wherein said tube is of polyethylene terephthalate, polypropylene or polystyrene.

7. The method of Claim 5 wherein said anticoagulant is heparin, sodium citrate or ethylenediaminetetraacetic acid.

8. A method for coating a blood collection tube comprising spraying a mist of a heparin salt in water unto the inside wall surface of a plastic tube and directing a flow of air over the misted surface to evaporate the water and leave a coating of particles of the salt on said surface.
